Europäisches Patentamt

European Patent Office

Office européen des brevets

(19)

(11) Publication number: **0 262 885**
**A2**

## (12) EUROPEAN PATENT APPLICATION

(21) Application number: **87308537.7**

(22) Date of filing: **28.09.87**

(51) Int. Cl.⁴: **A 61 K 7/06**
**A 01 N 53/00**

(30) Priority: **29.09.86 US 912119**

(43) Date of publication of application:
**06.04.88 Bulletin 88/14**

(84) Designated Contracting States: **DE FR GB IT**

(71) Applicant: **DEL LABORATORIES INCORPORATED**
**565 Broad Hollow Road**
**Farmingdale New York 11735 (US)**

(72) Inventor: **Gordon, Harry W.**
**1832 Cornelius Avenue**
**Wantaugh New York 11793 (US)**

**Guerrero, Angel**
**1921 Avenue G**
**Brooklyn New York 11230 (US)**

(74) Representative: **Ritter, Stephen David et al**
**Mathys & Squire 10 Fleet Street**
**London EC4Y 1AY (GB)**

(54) Pediculicidal/ovicidal shampoo composition.

(57) Pediculicidal/ovicidal liquid shampoo compositions comprise pyrethrins and a pyrethrin synergist in a shampoo vehicle together with a primary aromatic alcohol, a short-chain aliphatic alcohol and a long-chain aliphatic alcohol as ovicidal activity enhancers. The compositions exhibit excellent ectoparasiticidal and ovicidal activity, particularly with respect to Pediculus humanus var capitis.

EP 0 262 885 A2

**Description**

## PEDICULICIDAL/OVICIDAL SHAMPOO COMPOSITION

### BACKGROUND OF THE INVENTION

1. Field of the Invention:

The present invention relates to shampoo compositions useful for the treatment of lice and lice egg infestations in the hair and scalp.

2. Description of the Prior Art:

Throughout history, human beings in all parts of the world have suffered from parasitical infestations, including infestations in the hair and scalp by head lice (Pediculus humanus var capitis). Many methods of treating such head louse infestations have been proposed and utilized for centuries including, for example, washing the head with strong soaps and lye and the utilization of kerosene to kill adult lice and their ova. In more recent times, various ectoparasiticides have been incorporated into creams, gels, shampoos and the like for application to human hair and scalp.

Some of the ectoparasiticides which have been utilized as pediculicidal treatments since the early 1950's and are commercially available today in various forms include lindane ($1\alpha$ ,$2\alpha$ ,$3\beta$ ,$4\alpha$ ,$5\alpha$ ,$6\beta$-hexachlorocyclo-hexane); malathion (mercaptosuccinic acid diethyl esters); and pyrethrins (chrysanthemin carboxylic esters of pyrethrolone), derived from certain variants of the chrysanthemum family. Compositions containing pyrethrins as active ingredients normally include in addition a pyrethrin synergist such as piperonyl butoxide which greatly enhances pediculicidal activity. See generally Journal of the American Medical Association, Vol. 247 No. 22, pp. 3103-05, (June 11, 1982), for a description of various pediculicidal agents and compositions.

One of the most serious drawbacks of the commonly utilized prescription and non-prescription compositions for treatment of head louse infestations is that these compositions are highly effective against the adult parasites but do not produce significant mortality rates among louse eggs. Hence, while one or two applications of the commercially available pediculicidal compositions may kill 100% of the ectoparasites, the large number of ova left intact may simply hatch after a period of time and produce recurring infestation.

Although pediculicidal compositions have been proposed in the prior art which allegedly have higher ovicidal activity than the popular, commercially available compositions -- e.g., the imidazoline toxicants disclosed in U.S. Patent No. 4,238,499 -- said parasiticides, while effective against adult and nymphal forms, have not been shown to have consistent, substantial ovicidal activity, and have also not been proven safe, effective and inexpensive enough for widespread use in treating common louse infestation.

Shampoos are the preferred form of therapeutic composition for treating head lice because of their ease of use and removal and the degree to which the active ingredients can be dispersed throughout the hair and scalp while washing. However, for a liquid shampoo composition to display significant ectoparsiticidal and ovicidal activities, it must either exert these effects within a very short time (i.e., the duration between lathering of the shampoo and rinsing it off) or leave a residue of active ingredients on the hair and scalp which resists washing off and enables long term activity. However, no shampoo composition has been developed in the prior art which displays a high degree of ectoparasiticidal and ovicidal activity while simultaneously being safe, pleasant and inexpensive to use.

### SUMMARY OF THE INVENTION

1. Objects of the Invention:

It is an object of the present invention to provide liquid shampoo compositions which have substantial ectoparasiticidal and ovicidal activity for use in treating head louse infestations in humans.

An additional object of the present invention is to provide compositions as described above which are safe, effective, pleasant to use and relatively inexpensive.

A further object of the present invention is to provide liquid shampoo compositions as described above which contain synergized pyrethrins as active ingredients but incorporate additional ingredients which give the compositions greatly enhanced ovicidal activity in comparison with prior art pyreth rin-based compositions.

Still another object of the present invention is to provide compositions as described above which incorporate a combination of aromatic and aliphatic alcohols to increase the ovicidal activity.

Yet a further object of the present invention is to provide compositions as described above which incorporate synergized pyrethrins and a combination of alcohols in a conventional shampoo vehicle.

2. Brief Description of the Invention:

In keeping with these objects and others which will become apparent hereinafter, the present invention resides, briefly stated, in pyrethrin-containing liquid shampoo compositions which comprise from about 2 to about 10% by weight of a primary aromatic alcohol, about 2 to about 20% by weight of a first aliphatic alcohol and about 2 to about 20% by weight of a second aliphatic alcohol The phrase "__ % by weight" used throughout this specification and the appended claims refers to the percentage of the weight of the total composition constituted by the given component or ingredient.

Preferred alpihatic alcohols for use in the present invention include saturated $C_2$ to $C_5$ alcohols as the first aliphatic alcohol, preferably isopropyl alcohol, and saturated $C_7$ to $C_{12}$ alcohols as the second aliphatic alcohol, most preferably n-decanol. The preferred primary aromatic alcohol for use in the compositions in the present invention is benzyl alcohol.

It has been discovered that the alcohols incorporated into the subject shampoo compositions act synergistically to yield high ovicidal mortality rates.

In addition to the active and/or subactive ingredients of the present invention, the novel compositions include a conventional shampoo vehicle, comprising, by way of example, deionized water, detergents, fragrance, preservatives, antioxidant agents and the like.

DETAILED DESCRIPTION OF THE INVENTION

Primary aromatic alcohols, principally benzyl alcohol, are commonly used in commercially available shampoos and other treatments for infestation of Pediculus humanus var capitis.

More recently, isopropyl alcohol has been disclosed in the patent and medical literature as a particularly desirable component in pediculicidal and ovicidal compositions because it appears to enhance the activity of certain insecticidal toxicants (see, e.g., U.S. Patent Nos. 4,179,504; 4,238,499). However, pediculicidal/ovicidal compositions incorporating both primary aromatic and lower aliphatic alcohols in combination, are not known in the prior art.

It has now been discovered that certain primary aromatic alcohols and lower aliphatic alcohols, when added in combination in suitable concentration ranges to liquid pediculicidal/ovicidal compositions, particularly shampoos, act synergistically to provide greatly enhanced ovicidal activity. Suprisingly, it appears that the ovicidal activity of synergized pyrethrins alone, for example, is relatively insignificant in comparison with the activity of such synergized pyrethrins plus aromatic alcohol/lower aliphatic alcohols combination. Most of the ovicidal activity exhibited by the compositions of the present invention is attributable to the alcoholic components, with the pyrethrins providing primarily ectoparasiticidal activity.

It has further been discovered that the addition of a relatively long chain ($C_7$ to $C_{12}$) saturated aliphatic alcohol to an aromatic/lower aliphatic alcohol combination further increases the ovicidal activity of the aforesaid combination. In particular, n-decanol, which has been disclosed in the literature as possibly useful as a mosquito larvicide (Transactions of the Royal Society of Tropical Medicine and Hygiene, Vol. 77, No. 1, pp. 35-38 (1983)) but has never been used or proposed for use as an ingredient in a pediculicidal/ovicidal composition, was found to significantly enhance the ovicidal activity of a benzyl alcohol/isopropyl alcohol combination.

The pediculicidal/ovicidal compositions of the present invention contain synergized pyrethrins as their principal active ingredient in a conventional shampoo vehicle which additionally comprises in combination about 2 to about 10% by weight of a primary aromatic alcohol, about 2 to about 20% by weight of a first aliphatic alcohol and about 2 to about 20% by weight of a second aliphatic alcohol. Preferred alcoholic constituents, as indicated above, are benzyl alcohol, isopropyl alcohol and n-decanol, with preferred concentration ranges of about 4 to about 6% for the benzyl alcohol, about 8 to about 12% for the isopropyl alcohol and about 2 to about 12% for the n-decanol.

The concentration of pyrethrins in the novel compositions of the present invention is from about 0.1 to about 1% by weight, with a preferred concentration range of about 0.3 to about 0.4% pyrethrins. The pyrethrins may be utilized in the form of a petroleum distillate solution, such as "PYROCIDE 175" (McLaughlin Gormley King Co., Minneapolis, Minnesota), which is 80% petroleum distillate and 20% pyrethrins.

In addition to the pyrethrins, it is desirable for the subject compositions to include pyrethrin synergists such as piperonyl butoxide, which has long been known to enhance the parasiticidal activity of the pyrethrins.

The conventional shampoo vehicles of the novel compositions may comprise any non-irritating, pleasant-smelling shampoo composition known in the cosmetics art which is compatible with the pyrethrins and the alcoholic components of the present invention. Such conventional shampoo vehicles may include, by way of example, deionized or purified water, detergents such as alkyl/aryl ether sulfates (e.g., Na+, Mg++ NH$_4$+), thickeners such as cellulose derivatives and/or poly(ethylene oxide) ethers, antioxidants such as butylated hydroxytoluene, non-ionic coupling agents such as phenoxypolyethoxy ethanols and suitable fragrance and colors. In general, any shampoo vehicle which is compatible with the pyrethrins and alcoholic components of the present invention, is non-irritating and provides good foaming and rinsing action is suitable for use in the subject liquid shampoo compositions.

The compositions of the present invention may be prepared by any suitable means known in the cosmetics, toiletries and pharmaceutical industries for producing alcohol-containing shampoos. One suitable technique for producing the subject compositions entails the preparation of four separate phases, and the subsequent gradual combination of the four phases into a single batch which is mixed until a clear homogeneous liquid is achieved. One specific, illustrative example of such method of preparation is set forth in Example 1 below.

The ovicidal/pediculicidal shampoos of the present invention are intended primarily for treatment of Pediculus humanus var capitis in humans, but are also useful in the treament of crab lice (Phthirus pubis), body lice (Pediculus humanus corporis) and their nits. The compositions are used by applying a sufficient quantity of the shampoo to thoroughly wet the hair and skin of the affected areas. The shampoo is then worked thoroughly into the hair and small quantities of water are added until a good lather forms. The hairy area is then shampooed and rinsed thoroughly. When the hair is dry, the remaining nits and nit shells may be removed by fine-tooth combing, by tweezers or by other mechanical means.

Pesticide laboratory testing comparing the shampoo compositions of the present invention with the most popular commercially available compositions, both prescription and non-prescription, has demonstrated that whereas all of the tested compositions exhibit a high degree of ectoparasiticidal activity, the novel compositions of the invention exhibit a suprisingly greater degree of ovicidal activity. The comparative experimental data will be set forth below in the Examples.

The following are specific illustrative examples of pediculicidal/ovicidal shampoo compositions in accordance with the present invention as well as comparative test data relating to the same. These examples are not intended to limit or restrict the scope of the invention in any way and should not be construed as identifying specific materials, concentration ranges or methods of preparation which must be utilized exclusively in order to practice the present invention.

EXAMPLE 1

Liquid Shampoo Composition
The following ingredients were combined into four phases, as more fully described below:

| Phase | Ingredient | Weight Lbs. | Oz. | % w/w |
|-------|------------|-------------|------|-------|
| A | Deionized water (Purified Water, USP) | 27 | 0.64 | 27.04 |
| | "HAMPENE NA2 USP" (Edetate Disodium USP) W.R. Grace Co. Nashua, New Hampshire | | 1.60 | .10 |
| | "POLYOX WSR" (Poly (ethylene oxide)) Union Carbide Danbury, Connecticut | 1 | 8.00 | 1.5 |
| B | "EPAL 10" (n-decanol) Ethyl Corporation Baton Rouge, Louisiana | 10 | | 10.0 |
| | "SUSTANE BHT" (butylated hydroxytoluene) UOP Process Division Des Plaines, Illinois | | 3.20 | .2 |
| C | "PREMIUM PYROCIDE 175" (pyrethrins petroleum distillate) McLaughlin Gormley King Co. Minneapolis, Minnesota | 1 | 10.56 | 1.66 |
| | Piperonyl Butoxide Tech. McLaughlin Gormley King Co. | 4 | | 4.0 |
| | Benzyl Alcohol NF Ruger Chemical Irvington, New Jersey | 5 | | 5.0 |

4

| Phase | Ingredient | Weight Lbs. | Oz. | % w/w |
|-------|-----------|-------------|-----|-------|
| | "TRITON X-100" (Octoxynol 9 NF) Rohm & Haas Philadelphia, Pennsylvania | 5 | | 5.0 |
| | Perfume Oil Carruba, Inc. Milford, Connecticut | 1 | | 1.0 |
| 10.0 | Isopropyl Alcohol USP Elco Solvents Corp. | 10 | | |
| D | "SIPON" detergent (ammonium lauryl ether sulfate) Alcolac, Inc. Baltimore, Maryland | 34 | 8.00 | 34.5 |

Phase A:
Purified water USP was charged into a suitably sized mixing vessel equipped with a mixer. The edetate disodium was added to the water while mixing at 100-200 rpm until it was completely dissolved. Mixing was continued while the poly(ethylene oxide) was added slowly but steadily into the mixing vessel while mixing at 200-400 rpm. The mixing was continued until a smooth, lump-free, dispersion was obtained. Mixing was then stopped and the vessel covered.

Phase B:
N-decanol was charged into a second suitably sized mixing vessel equipped with a mixer. Butylated hydroxytoluene was added while mixing at 100-200 rpm until a sparkling clear solution was achieved. The mixing was then stopped and the vessel covered.

Phase C:
The pyrethrins were charged into a third mixing vessel equipped with a mixer. Piperonyl butoxide, benzyl alcohol, Octoxynol 9, fragrance and isopropyl alcohol were added one at a time to the pyrethrins while mixing slowly (25-75 rpm) until a clear homogeneous liquid was achieved.
Phase B was added to Phase C while mixing (25-75rpm) until a clear homogeneous liquid was obtained.

Phase D:
The ammonium lauryl ether sulfate detergent was added to Phase C while mixing until a homogeneous liquid was achieved, and mixing continued at 50-75 rpm.
While Phase C was mixed at 50-75 rpm, phase A was added in small portions. Mixing was continued until a homogeneous final product was obtained.

EXAMPLE 2

Comparative Pediculicidal and Ovicidal Potencies
The composition produced according to Example 1 was tested for pediculicidal/ovicidal activity, and the results of that testing were compared to the test results of a number of other formulations, described below:
Formula 1: The composition prepared according to Example 1.
Formula 2: A compositon substantially the same as Formula 1 but with no alcohols.
Formula 3: A composition substantially the same as Formula 2 but with 5% benzyl alcohol added.
Formula 4: A composition substantially the same as Formula 3 but containing 10% isopropyl alcohol in addition to 5% benzyl alcohol.
Formula 5: "KWELL Shampoo, manufactured by Reed and Carnrick, Piscataway, New Jersey. "KWELL" contains as its active ingredient 1% lindane USP in a base containing polyoxyethylene sorbitan monostearate, triethanolamine lauryl sulfate, acetone and purified water.
Formula 6: "RID" manufactured by Pfizer, Inc., New York, New York. "RID" contains as its active

5

ingredients 0.3% pyrethrins, 3.0% piperonyl butoxide, 1.2% petroleum distillate and 2.4% benzyl alcohol.

Formula 7: "R & C" Shampoo manufactured by Reed & Carnrick, Piscataway, New Jersey. "R&C" contains the identical active ingredients in the same concentrations as "RID" but without benzyl alcohol.

Formula 8: "A-200 PYRINATE" Shampoo manufactured by USV Development Corp., Manati, Puerto Rico. "A-200 PYRINATE" contains as its active ingredients 0.17% pyrethrins and 2% piperonyl butoxide in a base including benzyl aclohol, butyl stearate, fragrance, Octoxynol-9, oleic acid, oleoresin parsley seed, petroleum distillate and water.

Four replicates of 25 or 30 louse adults and four replicates of 25 or 30 louse eggs were immersed for 10 minutes in each of the above compositions, rinsed, washed and incubated. All of the compositions provided excellent control of louse adults with substantially 100% mortality at 1 and 24 hours post treatment. The mortalities among the eggs, however, were as follows:

TABLE 1

| Formula | 1 | 2 | 3 | 4 | 5 | 6 | 7 | 8 |
|---|---|---|---|---|---|---|---|---|
| Egg Mortality Rate (%) | 62.2* | 5.3 | 28.8 | 38.3 | 18.4* | 10.6* | 45.4* | 25.1* |

*These results represent averages of three separate ovicidal activity tests.

Hence, it was demonstrated that the composition of the present invention (Formula 1), containing the novel synergistic combination of an aromatic alcohol and two aliphatic alcohols, exhibited suprisingly and substantially greater ovicidal activity than the most popular commercially available compositions and experimental compositions (Formulas 2-4) lacking one or more of the alcoholic constituents.

The liquid shampoo compositions of the present invention are easy and inexpensive to formulate and produce in commercial quantities. They exhibit low toxicity and are safe and pleasant to use. By providing substantially increased ovicidal activity in comparison with prior art compositions, the shampoos of the invention can reduce the frequency and duration of treatments for louse infestation by ensuring that fewer eggs survive to produce a new ectoparasitic population in situ.

It will thus be seen that there are provided compositions which achieve the various objects of the invention and which are well adapted to meet the conditions of practical use. As various possible embodiments might be made in the embodiments above set forth, it is to be understood that all matters herein described are to be interpreted as illustrative and not in a limiting sense.

What is claimed as new and desired to be protected by Letters Patent is set forth in the appended claims.

**Claims**

1. A pyrethrin containing ovicidal/pediculicidal liquid shampoo composition comprising about 2 to about 10% by weight of a primary aromatic alcohol, about 2 to about 20% by weight of a first aliphatic alcohol and about 2 to about 20% by weight of a second aliphatic alcohol.

2. A composition according to claim 1 wherein said first aliphatic alcohol contains 2 to 5 carbon atoms per molecule and said second aliphatic alcohol contains 7 to 12 carbon atoms per molecule.

3. A composition according to claim 1 wherein said aromatic alcohol is benzyl alcohol.

4. A composition according to claim 2 wherein said first aliphatic alcohol is isopropyl alcohol and said second aliphatic alcohol is n-decanol.

5. A composition according to claim 1 which comprises about 4 to about 6% by weight of said aromatic alcohol, about 8 to about 12% by weight of said first aliphatic alcohol and about 2 to about 12% by weight of second aliphatic alcohol.

6. A composition acccording to claim 5 which comprises about 5% benzyl alcohol, about 10% isopropyl alcohol and about 10% n-decanol by weight.

7. A composition according to claim 1 which comprises about 0.1% to about 1% by weight pyrethrins.

8. A composition according to claim 7 which additionally comprises about 2 to about 6% by weight of at least one pyrethrin synergist.

9. A composition according to claim 8 wherein said pyrethrin synergist is piperonyl butoxide.